# EUROPEAN PATENT SPECIFICATION

(11) **EP 0 518 519 B1**
(45) Date of publication and mention of the grant of the patent: **08.05.1996**
(21) Application number: 92304749.2
(22) Date of filing: 27.05.1992
(51) Int. Cl.: A61F 5/445

(54) **Medico-surgical and sanitary articles and materials**
Medizinisch-chirurgische Hygieneartikel und Materialien
Articles et matériaux médico-chirurgicaux et hygiéniques

(30) Priority: 14.06.1991 GB 9112912
(43) Date of publication of application: 16.12.1992
(73) Proprietor: Smiths Industries Public Limited Company, London, NW11 8DS (GB)
(72) Inventor: Whiteside, Neil Adrian, Steyning, Sussex, BN44 3ME (GB)
(74) Representative: Flint, Jonathan McNeill

(56) References cited:
- EP-A- 0 142 950
- FR-A- 2 603 189
- GB-A- 2 064 333
- GB-A- 2 083 762
- GB-A- 2 185 404

## Description

This invention relates to w.c. disposable articles of the kind that can be disposed of in a w.c. by adding alkali to water in the w.c. pan.

The invention is more particularly concerned with ostomy or urine incontinence bags and sanitary articles such as nappies, sanitary towels and the like. The invention is also concerned with the materials from which such articles can be made.

Attempts have been made recently to develop ostomy and urine bags which can be disposed of by flushing in a w.c., to avoid the need to make special disposal arrangements which can be inconvenient, embarrassing and unhygenic.

W.C. disposable bags have been proposed in the literature which have an outer water-soluble or dispersable layer and an inner water-resistant layer. The outer layer provides mechanical support for the inner layer so that, when the bag is dropped into turbulent water in a w.c. pan, the outer layer is quickly broken up. The inner layer prevents the contents of the bag attacking the outer layer in use but, once the outer layer is broken up on disposal, the inner layer does not have sufficient mechanical strength in itself to cause blockage on flushing the w.c. An example of such a bag is described in GB 2083762B. The only w.c. disposable bag presently available is the Symphony bag sold by Eschmann Bros. & Walsh Limited, England (Symphony is a Registered Trade Mark of Eschmann Bros. & Walsh Limited).

Although such bags can be used satisfactorily, the fact that the outer layer is damaged by contact with water means that the user has to take special precautions to ensure that the outside of the bag does not become wet. This can be especially inconvenient with bags which are worn long-term, for two or more days, such as is usually the case with ileostomy bags. The use of such bags can make washing difficult and prevents the user swimming.

An alternative form of bag is described in EP 0142950A which is made of 3-hydroxybutyrate film either in a laminate with a water soluble film as an outer layer or entirely from 3-hydroxybutyrate. Such a material remains intact when in contact with water or body waste, but is broken up if the pH is raised to about 12. The bag described is disposed of by adding a base material to the contents of the bag so as to raise the pH of the contents to at least 12 so that it breaks up when agitated in a w.c. pan. The laminated construction would not avoid the disadvantages referred to above of having to keep the outside of the bag dry. Furthermore, 3-hydroxybutyrate does not provide sufficient odour barrier properties to be useful in a practical bag.

A further alkali-disposable bag is described in GB 2195919B. The walls of this bag have a central layer of polyvinyl alcohol, an inner layer of a blend of polyvinylidene chloride acrylonitrile copolymer with carboxylated acrylic copolymer, and an outer layer of two or more coatings of carboxylated acrylic acid. This bag can be disposed of in a w.c. by adding an alkali to the water in the pan. The material proposed for the inner layer combines the alkali-solubility of carboxylated acrylic copolymer with the high odour barrier properties of polyvinylidene chloride acrylonitrile copolymer, the blend being water resistant. However, in practice it has been found difficult to produce an inner layer which has sufficiently high odour barrier properties whilst also being broken up quickly in alkali. Furthermore, the polyvinyl alcohol central layer makes it difficult to coat with the inner layer because the high resistance of polyvinyl alcohol to organic solvents produces a weak interply adhesion. Attempts to use an aqueous based coating material have not been entirely satisfactory either because the polyvinyl alcohol abstracts the water from the coating too quickly to enable a high quality film to be produced. Although it is possible to produce bags according to GB 2195919B that will function, their speed of disposability and odour barrier properties are not as good as would be desired. This patent specification also refers briefly to the possibility that a two ply construction could be used, the outer ply being water insoluble but alkali dispersable, and the inner layer being of a different material which is water resistant. No disclosure of the composition of the materials that would be suitable is given.

It should be appreciated that there are several important criteria that must be satisfied by an ostomy bag. Firstly, it must be highly odour proof over the entire period for which it is worn and in all circumstances of varying temperature and humidity etc. Secondly, the bag must be inconspicuous from outside the wearer's clothing. For this reason, the bag material must not rustle or make other noises when the wearer moves. The bag should also be thin and flexible so that it conforms to the wearer's anatomy without producing bulges or ridges beneath the clothing. Thirdly, the bag must be reliable and secure so that the wearer can have high confidence that the bag will not tear or come apart at the edge seal during use. Fourthly, the bag must be affordable, which means that it must be able to be made at low cost.

Where a bag is also required to be w.c. disposable, this adds further difficulties to the choice of materials and manufacturing techniques, since the bag must possess all the above properties whilst also being capable of being disposed of by flushing in a w.c. The problems are further compounded if the bag must be resistant to water so that it can be worn safely in wet conditions. The selection of appropriate combinations of different materials that enables a bag to be produced which satisfies these requirements requires considerable skill and experiment.

It is an object of the present invention to provide improved medico-surgical and sanitary articles and materials.

According to one aspect of the present invention there is provided a w.c. disposable article of the above-specified kind, which article is made substantially from a material comprising a first layer substantially entirely of alkali-soluble carboxylated acrylic polymer and a second layer of alkali-resistant polyvinylidene chloride on one side of the first layer so that the layer of carboxylated acrylic polymer is broken up or dispersed by the water to which the alkali has been added, and wherin the thickness of the polyvinylidene chloride layer is sufficient to reduce odour transmission through the material to an acceptable level but is insufficient to impede flushing of the article after break up or dispersion of the layer of carboxylated acrylic polymer.

The layer of carboxylated acrylic polymer, in this way, provides the main structural support of the article whereas the polyvinylidene chloride layer provides the main odour barrier. It has been found that, by forming one layer of polyvinylidene chloride the high odour barrier properties of polyvinylidene chloride enable the layer to be made sufficiently thin that it will not impede flushing after breakup or dispersal of the other layer. In this way, therefore, it is unnecessary for both layers to be alkali soluble, a factor that has been previously prevented acceptable bags being made.

The second layer is preferably applied by coating on the first layer and may be an aqueous coating.

By coating in an aqueous base, it is possible to produce very good adhesion to the layer of carboxylated acrylic polymer and also to produce a very thin layer which is readily broken up once the other layer has been broken up or dispersed.

The first layer may be approximately 100 micron thick and the second layer be approximately 2 to 3 micron thick.

The article is preferably a medico-surgical collection bag, the first layer being presented externally to water in the w.c. pan to which the alkali has been added, the second layer being presented inwardly to the contents of the bag. The bag may comprise two sheets of material sealed together around their periphery by heat sealing the respective second layers to one another.

A w.c. disposable ostomy bag, in accordance with the present invention, and its method of manufacture will now be described, by way of example, with reference to the accompanying drawings, in which:
- Figure 1: is a front elevation view of the ostomy bag;
- Figure 2: is a sectional side elevation along the line II - II of Figure 1;
- Figure 3: shows a part of Figure 2 to an enlarged scale; and
- Figure 4: illustrates disposal of the bag.

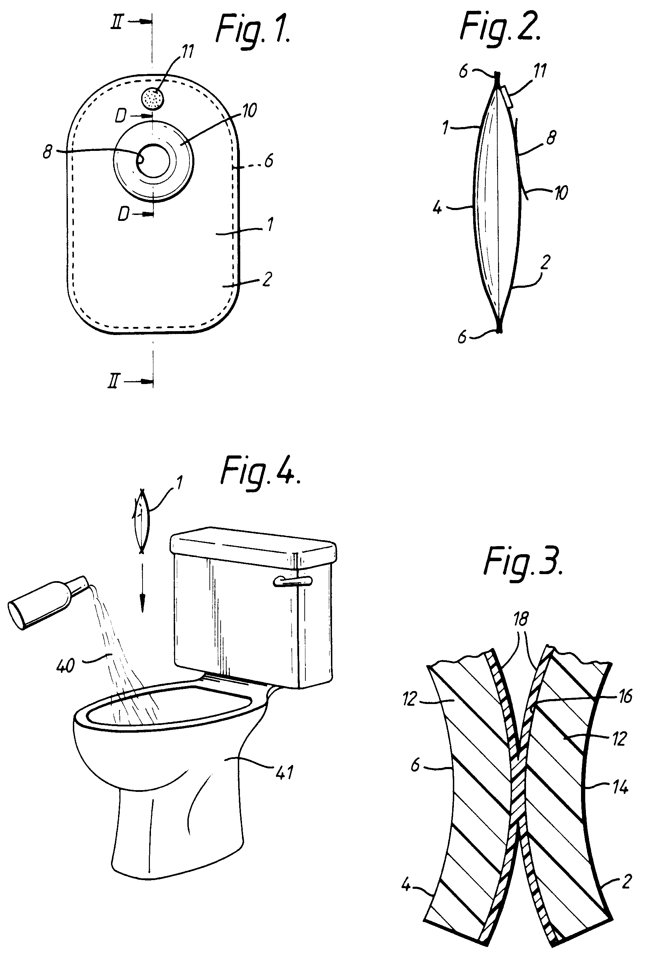

With reference to Figures 1 to 3, the ostomy bag 1 is of conventional shape and comprises two sheets 2 and 4 of the same material heat sealed together around their outer edge 6. The sheet 2, which is in use towards the body of the wearer, has an orifice 8 forming an opening to the bag. An adhesive flange 10 is secured to the sheet 2 around the orifice 8 and this serves to secure the bag to the user's skin around the stoma so that fecal matter is discharged into the bag. A filtered vent 11 is located towards the top of the bag 1.

The material from which both sheets 2 and 4 are made is shown in greater detail in Figure 3. Figure 3 does not show the relative thicknesses of the layers to correct scale. Each sheet comprises a continuous, imperforate layer 12 of carboxylated acrylic polymer of the kind sold by Belland AG under Grade No. FBC2620. Typically, the layer 12 is about 100 micron thick. One surface 14 of the layer is presented externally of the bag. On the opposite, internal surface 16, the layer 12 has a coating in the form of a continuous, imperforate layer 18 of polyvinylidene chloride which is about 2-3 micron thick, that is, thin compared with the layer 12. The layer 18 is presented inwardly to the contents of the bag on both sheets 2 and 4. Because polyvinylidene chloride provides a very effective odour barrier, it is only necessary to use a very thin layer, even though the layer 12 of carboxylated acrylic polymer has very poor odour barrier properties.

The material forming the sheets 2 and 4 is made by taking a layer 12 of carboxylated acrylic polymer and coating one side 16 with an aqueous-based polyvinylidene chloride such as Viclan VL874 sold by ICI. The coating is performed by gravure printing but could alternatively be carried out by other conventional coating techniques such as meyer bar, air knife or coextrusion. Although carboxylated acrylic polymer is resistant to water at normal pH values, it does have a good affinity for water with the result that an aqueous-based coating produces excellent film formation and interply adhesion. Instead of coating, it may be possible to form the layer of polyvinylidene chloride by other techniques.

In order to reduce water penetration into the layer 12 it may be coated on the side opposite the layer 18 with a thin coating of a blend of carboxylated acrylic polymer with polyvinylidene chloride, the blend comprising approximately 9 parts of carboxylated acrylic polymer to 1 part of polyvinylidene chloride. In this modification, the externally presented surface is still substantially entirely of carboxylated acrylic polymer.

After coating the layer 12 it is allowed to dry so that it can be cut to form the sheets 2 and 4. The sheets are then placed with their polyvinylidene chloride layers 18 facing one another and heat sealed around their outer edge 6. The good interply adhesion between the layers 12 and 18 ensures that the weld strength of the seal around the edge 6 of the bag is high. The flange 10 may then be attached by welding or adhesive.

The bag 1 is used in the usual way. The outer layer 12 provides the main structural support for the bag and its contents, whereas the inner layer 18 provides very little structural support. The inner layer 18, however, ensures that the level of odour that can be transmitted through the wall of the bag is reduced to a level that is acceptable to the user while the bag is in use (typically for about 12 hours) and in all normal situations of use involving variations in temperature and humidity. The nature of the outer layer 12 and the coating material forming the inner layer 18, ensure that the coating produces a high quality film with very few pin holes or imperfections. This is of prime importance in reducing odour transmission because the outer layer 12 provides substantially no odour barrier and any passage through the inner layer 18 would, therefore, allow escape of odour. The fact that the inner layer 18 is solely of polyvinylidene chloride means that its odour barrier properties are not compromised by other additives. The material of the sheets 2 and 4 is also flexible and noise free so that the bag will lie flat and remain inconspicuous under clothing. Because the outer layer 12 is resistant to water at neutral pH, the bag can be worn safely while washing, swimming or undertaking similar activities where the outside of the bag may become wet.

When the bag is full, it is disposed of in a conventional w.c. in the manner described below with reference to Figure 4.

The user first adds a quantity of a chemical 40 to the water in the w.c. pan 41 which is sufficient to raise the pH of the water to about 10. The preferred chemical is a mixture of triethanolamine (a water-soluble alkali) and a surfactant such as an ionic surfactant containing sodium dioctyl sulphosuccinate (eg Aerosol OT75 - Aerosol is a Registered Trade Mark of Cyanamid) in the ratio 5:1 by weight. The pH of this composition is 11.5 and, when diluted to 0.5% weight-weight in water, it has a pH of 10 at 15 degrees Centigrade. The undiluted additive is, therefore, not too active to be dangerous to the user but needs only to be added in relatively small quantities to the w.c. pan to be effective. The alkaline mixture is preferably in the form of a liquid which may be added to the w.c. pan from a bottle, syringe, sachet or similar container. Alternatively, alkalis in the form of powders or tablets can be used which are dissolved on contact with water in the w.c. pan.

The bag 1 is removed from the body and closed by folding the adhesive flange 10 in half about its vertical diameter D; excess air is expelled by squeezing through the vent 11. The bag 1 is then dropped into the w.c. pan 41 so that the outer surface of the bag is contacted by the alkali and water mixture. It will be appreciated that, a large proportion of the bag surface quickly becomes wetted by the surfactant and alkali which starts to break up the outer layer 12. After a few minutes in the pan 41, the outer layer 12 is dissolved or dispered by the action of the alkali.

The inner layer 18 is not affected by either water or alkali but it is so thin that, once it loses the structural support of the outer layer 12 it has insufficient strength by itself to present an obstacle to flushing.

Flushing of the w.c. agitates the water in the pan 41 helping further to break up the inner layer 18 or force it into more intimate contact with the bag contents. Any residual gas in the bag 1 escapes through the vent 11 or through ruptures in the bag as the w.c. is flushed, thereby allowing the contents of the bag and the remains of the bag itself to be flushed away without blockage.

The flange 10 is w.c. disposable and, in this respect may be of a material that becomes limp on contact with water and is of a suitable size that it is readily flushed away. For additional security, the flange could be water resistant but alkali disposable.

It will be appreciated that the alkali 40 can be added to the w.c. 41 after, or at the same time as, adding the bag 1.

The sheet material described above can be used for other w.c. disposable articles such as sanitary towels, nappies and bed pan liners. For some of these articles, it may be necessary to secure the material to a layer of absorbent material which would, in use, be placed against the skin.

## Claims

1. A w.c. disposable article that can be disposed of in a w.c. by adding alkali to water in the w.c. pan, which article (1) is made substantially from a material comprising a first layer (12) substantially entirely of alkali-soluble carboxylated acrylic polymer and a second layer (18) of alkali-resistant polyvinylidene chloride on one side of the first layer (12) so that the layer of carboxylated acrylic polymer is broken up or dispersed by the water to which the alkali has been added, and wherein the thickness of the polyvinylene chloride layer (18) is sufficient to reduce odour transmission through the material to an acceptable level but is insufficient to impede flushing of the article (1) after break up or dispersion of the layer (12) of carboxylated acrylic polymer.

2. An article according to Claim 1, characterised in that the second layer (18) is a coating on the first layer (12).

3. An article according to Claim 2, characterised in that the second layer (18) is an aqueous coating on the first layer (12).

4. An article according to any one of the preceding claims, characterised in that the first layer (12) is approximately 100 micron thick.

5. An article according to any one of the preceding claims, characterised in that the second layer (18) is approximately 2 to 3 micron thick.

6. An article according to any one of the preceding claims in which the article is a medico-surgical collection bag, characterised in that the first layer (12) is presented externally to water in the w.c. pan (41) to which the alkali (40) has been added, and that the second layer (18) is presented inwardly to the contents of the bag (1).

7. An article according to Claim 6, characterised in that the bag (1) comprises two sheets (2 and 4) of the material sealed together around their periphery (6) by heat sealing the respective second layers (18) to one another.

## Patentansprüche

1. Klosettentsorgbarer Artikel zur Entsorgung über ein Klosett durch Hinzufügung von Alkali zu Wasser in der Klosettschüssel, welcher im wesentlichen aus einem Material (1) gefertigt ist mit einer ersten Schicht (12), die im wesentlichen vollständig aus einem alkalilöslichen carboxylierten Acrylpolymer besteht und einer zweiten Schicht (18) aus alkaliresistentem Polyvinylidenchlorid auf einer Seite der ersten Schicht (12), so daß die Schicht aus carboxyliertem Acrylpolymer von dem Wasser, zu dem das Alkali hinzugefügt wurde, aufgebrochen oder gelöst wird, und wobei die Dicke der Polyvinylidenchloridschicht (18) ausreichend groß ist, um den Geruchsdurchgang durch das Material auf einen akzeptablen Wert zu reduzieren, jedoch nicht groß genug, um das Herunterspülen des Artikels (1) nach dem Aufbrechen oder der Lösung der Schicht (12) aus carboxyliertem Acrylpolymer zu behindern.

2. Artikel nach Anspruch 1, **dadurch gekennzeichnet**, daß die zweite Schicht (18) eine Beschichtung auf der ersten Schicht (12) ist.

3. Artikel nach Anspruch 2, **dadurch gekennzeichnet**, daß die zweite Schicht (18) eine wässrige Beschichtung auf der ersten Schicht (12) ist.

4. Artikel nach einem der voranstehenden Ansprüche, **dadurch gekennzeichnet**, daß die erste Schicht (12) etwa 100 µm dick ist.

5. Artikel nach einem der voranstehenden Ansprüche, **dadurch gekennzeichnet**, daß die zweite Schicht (18) etwa 2 bis 3 µm dick ist.

6. Artikal nach einem der voranstehenden Ansprüche, wobei der Artikel ein medizinisch-chirurgischer Sammelbeutel ist, **dadurch gekennzeichnet**, daß die erste Schicht (12) nach außen dem Wasser in der Klosettschüssel (41) zugewandt ist, zu welchem das Alkali (40) hinzugefügt wurde und die zweite Schicht (18) nach innen dem Inhalt des Beutels (1) zugewandt ist.

7. Artikel nach Anspruch 6, **dadurch gekennzeichnet**, daß der Beutel (1) zwei Bögen (2 und 4) des Materials aufweist, welche entlang ihres Umfangs (6) durch Hitzeverschweißung der entsprechenden zweiten Schichten (18) miteinander verschweißt sind.

## Revendications

1. Article jetable pour WC, qui peut être évacué dans un WC, par addition d'alcalis à l'eau d'une cuvette de WC, cet article (1) étant fait essentiellement en un matériau comprenant une première couche (12) en un polymère acrylique carboxylé entièrement soluble aux alcalis et une seconde couche (18) de chlorure de polyvinylidène résistant aux alcalis, disposée sur un côté de la première couche (12) de telle manière que la couche de polymère acrylique carboxylé est brisée ou dispersée par l'eau à laquelle les alcalis ont été ajoutés, et dans lequel l'épaisseur de la couche (18) de chlorure de polyvinylidène est suffisante pour réduire la transmission d'odeurs à travers le matériau jusqu'à un niveau acceptable, mais est insuffisante pour empêcher l'évacuation de l'article (1) après rupture ou dispersion de la couche (12) de polymère acrylique carboxylé.

2. Article selon la revendication 1, caractérisé en ce que la seconde couche (18) est un revêtement de la première couche (12).

3. Article selon la revendication 2, caractérisé en ce que la seconde couche (18) est un revêtement aqueux de la première couche (12).

4. Article selon l'une quelconque des revendications précédentes, caractérisé en ce que la première couche (12) a une épaisseur d'environ 100 microns.

5. Article selon l'une quelconque des revendications précédentes, caractérisé en ce que la seconde couche (18) a une épaisseur d'environ 2 à 3 microns.

6. Article selon l'une quelconque des revendications précédentes, cet article étant une poche collectrice médico-chirurgicale, caractérisé en ce que la première couche (12) est mise en contact extérieurement avec l'eau d'une cuvette de WC (41) à laquelle on a ajouté des alcalis (40) et en ce que la seconde couche (18) est en contact avec le contenu intérieur de la poche (1).

7. Article selon la revendication 6, caractérisé en ce que la poche (1) comporte deux feuilles (2 et 4) du matériau, soudées l'une à l'autre à leur périphérie (6) par scellement à chaud des secondes couches respectives (18) l'une contre l'autre.
